# EUROPEAN PATENT APPLICATION

(11) **EP 3 741 854 A1**
(43) Date of publication of application: **25.11.2020**
(21) Application number: 19741228.1
(22) Date of filing: 16.01.2019
(51) Int. Cl.: C12N 15/11, A61K 49/00, A61L 31/02, A61L 31/04, A61L 31/10, A61L 31/12, A61L 31/14, C07K 1/13, C07K 7/08, C07K 16/00

(54) **METAL-BINDING PEPTIDE AND USE THEREOF**

(30) Priority: 17.01.2018 JP 2018005452
(71) Applicant: THE JIKEI UNIVERSITY SCHOOL OF MEDICINE, Tokyo 105-8461 (JP)
(72) Inventor: TERAMURA Yuji, Tokyo 113-8654 (JP); AZUMA Tomoyuki, Tokyo 113-8654 (JP); YOSHIHARA Akifumi, Tokyo 113-8654 (JP); KODAMA Tomonobu, Tokyo 105-8461 (JP); MURAYAMA Yuichi, Tokyo 105-8461 (JP)
(74) Representative: Vossius & Partner Patentanwälte Rechtsanwälte mbB
(86) International application number: PCT/JP2019/001006
(87) International publication number: WO 2019/142800

(57) **Abstract**

The present invention is intended to provide a peptide that selectively binds to a metal surface. The present invention relates to a peptide consisting of an amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, or SEQ ID NO: 21, or a peptide substantially identical to the aforementioned peptide, and a method for detecting a metal surface of a medical device, using these peptides.

SEQUENCE LISTING

<110>  The University of Tokyo
       The Jikei University School of Medicine

<120>  Metal-binding peptide and use thereof

<130>  TPC0253UVT

<150>  JP2018-005452
<151>  2018-01-17

<160>  21

<170>  PatentIn version 3.5

<210>  1
<211>  12
<212>  PRT
<213>  Artificial Sequence

<220>
<223>  Synthetic peptide

<400>  1

Met Lys Ala His His Ser Gln Leu Tyr Pro Arg His
1               5                   10


<210>  2
<211>  12
<212>  PRT
<213>  Artificial Sequence

<220>
<223>  HRPYLQSHHAKMGGGC

<400>  2

His Arg Pro Tyr Leu Gln Ser His His Ala Lys Met
1               5                   10


<210>  3
<211>  12
<212>  PRT
<213>  Artificial Sequence

<220>
<223>  Synthetic peptide

<400>  3

Ser Gly His Phe Phe Met Lys Asp His Trp Asp Val
1               5                   10


<210>  4
<211>  12
<212>  PRT
<213>  Artificial Sequence

<220>
<223>  Synthetic peptide

<400>  4

Val Asp Trp His Asp Lys Met Phe Phe His Gly Ser
1               5                   10


<210>  5
<211>  12
<212>  PRT
<213>  Artificial Sequence

<220>
<223>  Synthetic peptide

<400>  5

His Ala Pro Asp Thr Ile Lys Arg Ser Leu Ala Met
1               5                   10


<210>  6
<211>  12
<212>  PRT
<213>  Artificial Sequence

<220>
<223>  Synthetic peptide

<400>  6

Asp Lys Asp Val Thr His Phe Leu Glu Arg Thr Arg
1               5                   10


<210>  7
<211>  12
<212>  PRT
<213>  Artificial Sequence

<220>
<223>  Synthetic peptide

<400>  7

Asn His His Met Met Pro Ala Trp Asn Val Lys His
1               5                   10


<210>  8
<211>  12
<212>  PRT
<213>  Artificial Sequence

<220>
<223>  Synthetic peptide

<400>  8

Asn Leu Gly Ala Glu Pro Gly Thr Pro Tyr Leu Val
1               5                   10

<210>  9
<211>  12
<212>  PRT
<213>  Artificial Sequence

<220>
<223>  Synthetic peptide

<400>  9

Tyr Gln Pro Ala Arg Glu His Arg Val Pro Ala Gly
1               5                   10


<210>  10
<211>  12
<212>  PRT
<213>  Artificial Sequence

<220>
<223>  Synthetic peptide

<400>  10

Thr Asn Asn Asn Thr Pro Ser Gln Met Gly Leu Ser
1               5                   10


<210>  11
<211>  12
<212>  PRT
<213>  Artificial Sequence

<220>
<223>  Synthetic peptide

<400>  11

Phe Gln Gln Ala Ser Gln Ala Arg Pro Ser Ser Pro
1               5                   10


<210>  12
<211>  12
<212>  PRT
<213>  Artificial Sequence

<220>
<223>  Synthetic peptide

<400>  12

Ala Asp Ser Asn His Ala Tyr Glu Arg Asp Ser Val
1               5                   10


<210>  13
<211>  12
<212>  PRT
<213>  Artificial Sequence

<220>
<223>  Synthetic peptide

<400>  13

Leu Gly Thr Lys Pro Ala Leu Ser Pro Thr Asn Phe
1               5                   10


<210>  14
<211>  12
<212>  PRT
<213>  Artificial Sequence

<220>
<223>  Synthetic peptide

<400>  14

Thr Leu Asn Val Pro Pro Ala Lys Arg Ser Leu Ser
1               5                   10


<210>  15
<211>  12
<212>  PRT
<213>  Artificial Sequence

<220>
<223>  Synthetic peptide

<400>  15

Ala Thr His Gly Pro Lys Gln Val Ser Ser Trp Thr
1               5                   10


<210>  16
<211>  12
<212>  PRT
<213>  Artificial Sequence

<220>
<223>  Synthetic peptide

<400>  16

Ala His Asn His Thr Pro Ile Lys Gln Lys Tyr Leu
1               5                   10


<210>  17
<211>  12
<212>  PRT
<213>  Artificial Sequence

<220>
<223>  Synthetic peptide

<400>  17

Leu Tyr Lys Gln Lys Ile Pro Thr His Asn His Ala
1               5                   10

<210>  18
<211>  12
<212>  PRT
<213>  Artificial Sequence

<220>
<223>  Synthetic peptide

<400>  18

Leu Thr Pro His Lys His His Lys His Leu His Ala
1               5                   10


<210>  19
<211>  12
<212>  PRT
<213>  Artificial Sequence

<220>
<223>  Synthetic peptide

<400>  19

Ala His Leu His Lys His His Lys His Pro Thr Cys
1               5                   10


<210>  20
<211>  12
<212>  PRT
<213>  Artificial Sequence

<220>
<223>  Synthetic peptide

<400>  20

Ser Ala Val Gln Trp Phe Glu Leu Asn Thr His Ala
1               5                   10


<210>  21
<211>  12
<212>  PRT
<213>  Artificial Sequence

<220>
<223>  Synthetic peptide

<400>  21

Ala His Thr Asn Leu Glu Phe Trp Gln Val Ala Ser
1               5                   10

## Description

### Technical Field

The present invention relates to a peptide that binds to a metal.

### Background Art

In recent years, for the treatment of cerebral aneurysm, ischemic heart disease and the like, intervention treatment using a stent or a coil, which is more minimally invasive than craniotomy or thoracotomy, has been widely performed. Because of such a method, burden on patients has been significantly reduced. However, since metallic stents or coils are poorly biocompatible, the intervention treatment has caused clinical problems regarding complications such as restenosis of blood vessels and stent thrombosis, which occur after the placement of these medical devices.

With regard to restenosis after intervention, with the use of drug-eluting stent (DES), neointimal formation has been suppressed, and thus, the in-stent restenosis rate has been significantly reduced.

In contrast, with regard to stent thrombosis, there have been reports regarding "late stent thrombosis (LST)" generated one or more months after stent placement and "very late stent thrombosis (VLST)" generated one or more years after stent placement, which had rarely occurred upon the use of metallic stents (Non Patent Literature 1). In addition, some cases have been reported, in which ischemic events occurred after stent placement, even in a single antiplatelet drug administration period (Non Patent Literature 2). Thus, new problems appeared regarding stent thrombosis.

At present, in order to prevent LST or VLST, dual antiplatelet therapy (DAPT) has become a mainstream, and a thienopyridine antiplatelet drug has been administered in combination with aspirin after the placement of DES. DAPT needs to be continued for a long period of time, but an appropriate administration period thereof has not yet been revealed. Moreover, regarding DAPT, the risk of bleeding has been significantly increased (Non Patent Literature 3), and also, the risk of death has tended to be increased (Non Patent Literature 4).

As mentioned above, in order to prevent stent thrombosis such as LST or VLST, DAPT is extremely effective and needs to be continued, at least, until the metal surface is covered with endothelial cells. However, a clear consensus has not yet been reached regarding the period at which the metal surface is covered with endothelial cells, and further, an effective means for examining whether or not the metal surface is covered with endothelial cells has not existed so far.

### Citation List

### Patent Literature

Patent Literature 1: US2010/0048637 A1
Patent Literature 2: US6,683,108 B1

### Non Patent Literature

Non Patent Literature 1: McFadden et al., Lancet 364: 1519-1521, 2004
Non Patent Literature 2: Ong et al., JACC 45: 947-953, 2005
Non Patent Literature 3: Mauri et al., N Engl J Med. 371: 2155-2166, 2014
Non Patent Literature 4: Kereiakes et al., JAMA. 313: 1113-1121, 2015
Non Patent Literature 5: Seker et al., Molecules 16: 1426-1451, 2011

### Summary of Invention

### Technical Problem

Under the aforementioned circumstance, the present invention is intended to detect the surface of a metallic medical device (for example, a stent, a coil for embolization, etc.) that is not covered with endothelial cells, by using a peptide.

Therefore, it is an object of the present invention to provide a peptide that selectively binds to the surface of a metal, in particular, the surface of a metallic medical device (a stent, a coil for embolization, etc.).

### Solution to Problem

The present inventors have conceived that if a detectable labeling substance-added peptide selectively bound to the surface of a metal such as a stent or a coil for embolization in blood, an uncovered surface of such a medical device placed in the body could be selectively detected. Hence, the inventors have attempted to prepare a peptide that selectively binds to the surface of a metallic medical device particularly placed in the blood vessel, such as a stent or a coil for embolization.

The present inventors have searched for a peptide having high affinity for a stent (Ni : Ti = 55% : 45%, STRYKER) or a coil for embolization (Pt : W = 92% : 8%, Boston Scientific) according to a phage display method (Non Patent Literature 5).

The present invention has been completed based on the aforementioned findings.

Specifically, the present invention includes the following (1) to (13).
(1) A peptide consisting of an amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15.
(2) A peptide of the following (a) or (b), which selectively binds to nickel and/or titanium:
   (a) a peptide consisting of an amino acid sequence comprising a substitution, deletion, insertion and/or addition of one or several amino acids, with respect to the amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15, or
   (b) a peptide consisting of an amino acid sequence having a sequence identity of 90% or more to the amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15.
(3) The peptide according to the above (1) or (2), to which a detectable labeling compound binds.
(4) A peptide consisting of an amino acid sequence as set forth in SEQ ID NO: 16, SEQ ID NO: 17, SEQ ID NO: 18, SEQ ID NO: 19, SEQ ID NO: 20, or SEQ ID NO: 21.
(5) A peptide of the following (a) or (b), which selectively binds to platinum and/or tungsten:
   (a) a peptide consisting of an amino acid sequence comprising a substitution, deletion, insertion and/or addition of one or several amino acids, with respect to the amino acid sequence as set forth in SEQ ID NO: 16, SEQ ID NO: 17, SEQ ID NO: 18, SEQ ID NO: 19, SEQ ID NO: 20, or SEQ ID NO: 21, or
   (b) a peptide consisting of an amino acid sequence having a sequence identity of 90% or more to the amino acid sequence as set forth in SEQ ID NO: 16, SEQ ID NO: 17, SEQ ID NO: 18, SEQ ID NO: 19, SEQ ID NO: 20, or SEQ ID NO: 21.
(6) The peptide according to the above (4) or (5), to which a detectable labeling compound binds.
(7) A peptide complex comprising two or more peptides selected from the group of the peptides according to the above (1), (2), and (3).
(8) A peptide complex comprising two or more peptides selected from the group of the peptides according to the above (4), (5), and (6).
(9) An antibody against the peptide according to any one of the above (1), (2), (4), or (5).
(10) A nucleic acid encoding the peptide according to any one of the above (1), (2), (4), or (5).
(11) A detection reagent for detecting a metal surface of a device made of nickel and/or titanium, wherein the detection reagent comprises the peptide selected from the group of the peptides according to the above (1), (2), and (3), and/or the peptide complex according to the above (7).
(12) A detection reagent for detecting a metal surface of a device made of platinum and/or tungsten, wherein the detection reagent comprises one or more peptides selected from the group of the peptides according to the above (4), (5), and (6), and/or the peptide complex according to the above (8).
(13) A metallic device, to which the peptide according to any one of the above (1) to (6) or the peptide complex according to the above (7) or (8) binds.

### Advantageous Effects of Invention

By using the peptide according to the present invention, a surface (metal surface) of a metallic medical device (for example, a stent, a coil for embolization, etc.) can be detected.

By using the peptide according to the present invention, a non-coated portion (for example, a surface portion that is not coated with vascular endothelial cells, etc.) of a metallic medical device (for example, a stent, a coil for embolization, etc.) placed in the body can be detected.

### Brief Description of Drawings

[Figure 1] Figure 1 includes photomicrographs taken when the fluorescently labeled peptides of the present invention were each allowed to bind to a stent and were then observed under a fluorescence microscope. "Untreated" indicates a photomicrograph of a stent that is not treated with the peptide.
[Figure 2] Figure 2 includes photomicrographs taken when the fluorescently labeled peptides of the present invention were each allowed to bind to a stent and were then observed under a fluorescence microscope. "Untreated" indicates a photomicrograph of a stent that is not treated with the peptide.
[Figure 3] Figure 3 includes photomicrographs taken when the fluorescently labeled peptides of the present invention were each allowed to bind to a stent and were then observed under a fluorescence microscope. "Untreated" indicates a photomicrograph of a stent that is not treated with the peptide.
[Figure 4] Figure 4 is a graph formed by digitizing the fluorescence intensities of fluorescence states observed in Figure 1 to Figure 3.
[Figure 5] Figure 5 includes photomicrographs taken when the fluorescently labeled peptides of the present invention were each allowed to bind to a coil for embolization and were then observed under a fluorescence microscope. "Untreated" indicates a photomicrograph of a coil for embolization that is not treated with the peptide.
[Figure 6] Figure 6 includes photomicrographs taken when the fluorescently labeled peptides of the present invention were each allowed to bind to a coil for embolization and were then observed under a fluorescence microscope. "Untreated" indicates a photomicrograph of a coil for embolization that is not treated with the peptide.
[Figure 7] Figure 7 is a graph formed by digitizing the fluorescence intensities of fluorescence states observed in Figure 5 and Figure 6.
[Figure 8] Figure 8 is a conceptual diagram showing a peptide complex formed by binding the peptide of the present invention to 4-branched polyethylene glycol (PEG).
[Figure 9] Figure 9 includes photomicrographs taken when a fluorescently labeled peptide complex (a conjugate of 4-branched PEG and peptides) was allowed to bind to a stent and was then observed under a fluorescence microscope. "Untreated" indicates a photomicrograph of a stent that is not treated with the peptide complex.
[Figure 10] Figure 10 includes photomicrographs taken when a fluorescently labeled peptide complex (a conjugate of 4-branched PEG and peptides) was allowed to bind to a stent and was then observed under a fluorescence microscope. "Untreated" indicates a photomicrograph of a stent that is not treated with the peptide complex.
[Figure 11] Figure 11 includes photomicrographs taken when a fluorescently labeled peptide complex (a conjugate of 4-branched PEG and peptides) was allowed to bind to a stent and was then observed under a fluorescence microscope. "Untreated" indicates a photomicrograph of a stent that is not treated with the peptide complex.
[Figure 12] Figure 12 is a graph formed by digitizing the fluorescence intensities of fluorescence states observed in Figure 9 to Figure 11.
[Figure 13] Figure 13 includes photomicrographs taken when a fluorescently labeled peptide complex (a conjugate of 4-branched PEG and peptides) was allowed to bind to a coil for embolization and was then observed under a fluorescence microscope. "Untreated" indicates a photomicrograph of a coil for embolization that is not treated with the peptide complex.
[Figure 14] Figure 14 is a graph formed by digitizing the fluorescence intensities of fluorescence states observed in Figure 13.
[Figure 15] Figure 15 includes photomicrographs taken when the fluorescently labeled peptides of the present invention were each allowed to bind to a stent that has been immersed in human plasma, and were then observed under a fluorescence microscope. As a control peptide, YT07 that does not bind to a stent was used.
[Figure 16] Figure 16 includes photomicrographs taken when the fluorescently labeled peptides of the present invention were each allowed to bind to a stent that has been immersed in human plasma, and were then observed under a fluorescence microscope. As a control peptide, YT07 that does not bind to a stent was used.
[Figure 17] Figure 17 is a graph formed by digitizing the fluorescence intensities of fluorescence states observed in Figure 15 and Figure 16.

### Description of Embodiments

A first embodiment of the present invention relates to a peptide consisting of an amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15, or a peptide substantially identical to the aforementioned peptide.

The peptide consisting of an amino acid sequence as set forth in each of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, and SEQ ID NO: 15, has an activity of selectively binding to devices made of nickel and/or titanium. In this context, the term "devices made of nickel and/or titanium" means devices produced from only nickel, only titanium, or an alloy of nickel and titanium (nickel-titanium alloy) as a raw material. The device is particularly preferably a medical device placed in a body, such as a stent (i.e., a medical device for extending tubular tissues or organs in a living body (e.g., blood vessel, trachea, esophagus, duodenum, large intestine, biliary tract, etc.) from the inside thereof, wherein the medical device is a mesh cylindrical device made of a metal), or a coil for embolization (i.e., a long and thin medical device made of a metal, which is used in the embolization of an aneurysm and the like). The composition of such a nickel-titanium alloy is not particularly limited, and in a case where the device is a medical device (in particular, a stent or a coil for embolization), the device produced from the nickel-titanium alloy may have any composition, as long as it has the properties of a shape memory alloy. As an example, the nickel-titanium alloy may comprise 50% to 60% of nickel and 40% to 50% of titanium.

In the first embodiment, the "peptide substantially identical to the peptide consisting of an amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15" is a peptide of the following (a) or (b), which selectively binds to nickel and/or titanium:
(a) a peptide consisting of an amino acid sequence comprising a substitution, deletion, insertion and/or addition of one or several amino acids, with respect to the amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15, or
(b) a peptide consisting of an amino acid sequence having a sequence identity of 90% or more to the amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15.

A second embodiment of the present invention relates to a peptide consisting of an amino acid sequence as set forth in SEQ ID NO: 16, SEQ ID NO: 17, SEQ ID NO: 18, SEQ ID NO: 19, SEQ ID NO: 20, or SEQ ID NO: 21, or a peptide substantially identical to the aforementioned peptide.

The peptide consisting of an amino acid sequence as set forth in each of SEQ ID NO: 16, SEQ ID NO: 17, SEQ ID NO: 18, SEQ ID NO: 19, SEQ ID NO: 20, and SEQ ID NO: 21, has an activity of selectively binding to devices made of platinum and/or tungsten. In this context, the term "devices made of platinum and/or tungsten" means devices produced from only platinum, only tungsten, or an alloy of platinum and tungsten (platinum-tungsten alloy) as a raw material. The device is particularly preferably a medical device placed in a body, such as a stent or a coil for embolization. The composition of such a platinum-tungsten alloy is not particularly limited, and in a case where the device is a medical device (in particular, a stent or a coil for embolization), the device produced from the platinum-tungsten alloy may have any composition, as long as it has the properties of a shape memory alloy. As an example, the platinum-tungsten alloy may comprise 85% to 100% of platinum and 0% to 25% of titanium.

In the second embodiment, the "peptide substantially identical to the peptide consisting of an amino acid sequence as set forth in SEQ ID NO: 16, SEQ ID NO: 17, SEQ ID NO: 18, SEQ ID NO: 19, SEQ ID NO: 20, or SEQ ID NO: 21" is a peptide of the following (a) or (b), which selectively binds to platinum and/or tungsten:
(a) a peptide consisting of an amino acid sequence comprising a substitution, deletion, insertion and/or addition of one or several amino acids, with respect to the amino acid sequence as set forth in SEQ ID NO: 16, SEQ ID NO: 17, SEQ ID NO: 18, SEQ ID NO: 19, SEQ ID NO: 20, or SEQ ID NO: 21, or
(b) a peptide consisting of an amino acid sequence having a sequence identity of 90% or more to the amino acid sequence as set forth in SEQ ID NO: 16, SEQ ID NO: 17, SEQ ID NO: 18, SEQ ID NO: 19, SEQ ID NO: 20, or SEQ ID NO: 21.

In the first embodiment and the second embodiment, in the case of using the expression "an amino acid sequence comprising a substitution, deletion, insertion and/or addition of one or several amino acids," the number of amino acids substituted, deleted, inserted, and/or added is not particularly limited, and for example, the number of such amino acids is preferably approximately 1, 2, or 3. In addition, in the present description, the sequence identity of the "amino acid sequence having a sequence identity of 90% or more" is not particularly limited, as long as the amino acid sequence is an amino acid sequence having a sequence identity of 90% or more. For example, the sequence identity may be 90% or more, 91% or more, 92% or more, 93% or more, 94% or more, 95% or more, 96% or more, 97% or more, 98% or more, or 99% or more.

To the peptides according to the first embodiment and the second embodiment of the present invention (hereinafter referred to as "the peptide of the present invention"), a detectable labeling substance may bind. Herein, the type of such a detectable labeling substance is not particularly limited, as long as the labeling substance can be detected *in vitro* or *in vivo* and signals from the labeling substance can be quantified. Examples of such a detectable labeling substance may include: fluorescent substances (e.g., chemical synthetic substances such as fluorescein, rhodamine, Cy dye, AlexaR Fluor, and HiLyte™ Fluor, and fluorescent proteins such as GFP and a derivative thereof, phycoerythrin (PE), and allophycocyanin (APC)); and radioactive labeling substances (e.g., ⁶⁷Ga, ^{99m}Tc, ¹¹¹In, ¹²³I, ²⁰¹TI, etc. for SPEC labeling, and ¹¹C, ¹³N, ¹⁸F, ¹⁵O, etc. for PET labeling). In particular, for detection of a peptide existing in a living body, (e.g., a labeled peptide binding to a stent or a coil for embolization placed in the body), a radioactive labeling substance is desirably used, and a radioactively labeled peptide can be detected and quantified according to a PET (positron emission tomography) method or a SPECT (single photon emission computed tomography) method.

Moreover, detection by MRI is also preferable. For instance, in such detection by MRI, the peptide can be labeled with gadolinium (Gd) or superparamagnetic iron oxide (SPIO) nanoparticles. Chelating agents for gadolinium include diethylenetriamine penta-acetic acid (DTPA) and 1,4,7,10-tetraazacyclododecane-1,4,7,10-tetraacetic acid (DOTA). With regard to a method of allowing gadolinium to bind to the peptide, Leon-Rodriguez et al., J. AM. CHEM. SOC. 124: 3514-3515 2002, etc. can be referred. In addition, superparamagnetic iron oxide nanoparticles can be directly bound to terminal cysteine according to chemical modification (see Egawa et al., Biomaterials 54: 158-167 2015, etc.).

The C-terminus of the peptide of the present invention is generally a carboxyl group (-COOH) or carboxylate (-COO-), and further, the carboxyl group may be chemically modified with an amide (-CONH₂), an ester (-COOR) or the like. Herein, examples of R in the ester may include an alkyl group containing 1 to 6 carbon atoms (e.g., methyl, ethyl, n-propyl, isopropyl or n-butyl), a cycloalkyl group containing 3 to 8 carbon atoms (e.g., cyclopentyl or cyclohexyl), an aryl group containing 1 to 6 carbon atoms (e.g., phenyl or α-naphthyl), a phenyl-C1-2 alkyl group (e.g., benzyl or phenethyl), and an α-naphthyl-C1-2 alkyl group (e.g., α-naphthylmethyl). A peptide having carboxyl groups in the peptide chain thereof as well as at the C-terminus thereof, wherein the carboxyl groups are amidated or esterified, is also included in the peptide of the present invention. In this case, examples of the ester may include the above-described esters.

Moreover, the N-terminus of the peptide of the present invention is generally an amino group (-NH₂), and this amino group may be chemically modified with, for example, an acyl group containing 1 to 6 carbon atoms, such as a formyl group or an acetyl group. What is more, a peptide whose N-terminal side is cleaved *in vivo* to generate glutamyl group, which is then converted to pyroglutamic acid, a peptide in which the substituent on the side chain of amino acid in a molecule thereof (e.g., -OH, -SH, an amino group, an imidazole group, an indole group, a guanidino group, etc.) is chemically modified with a suitable functional group (e.g., a formyl group, an acetyl group, etc.), and a peptide to which a sugar chain binds are also included in the peptide of the present invention.

Furthermore, the peptide of the present invention also includes those constituted by non-natural peptide bonds such as, for example, ketomethylene or thioamide.

The peptide of the present invention can be prepared by methods well known in the present technical field, for example, genetically engineering methods and chemical peptide synthetic methods (solid phase methods or liquid phase methods).

In the case of chemically synthesizing a peptide, a portion of a peptide constituting the peptide of the present invention or amino acid and a residual portion are condensed, and if a product has a protective group, the protective group is dissociated from the product, so as to produce a peptide of interest. After completion of the synthetic reaction, ordinary purification methods such as, for example, solvent extraction, distillation, column chromatography, high performance liquid chromatography or recrystallization, are combined with one another and are applied to the synthesized peptide, so that the partial peptide of the present invention can be isolated and purified.

Further, the present invention includes a nucleic acid encoding the peptide of the present invention. In this context, the nucleic acid may be either DNA or RNA, or may also be either single-stranded or double-stranded. This nucleic acid can be used to synthesize the peptide of the present invention. The peptide of the present invention can be easily synthesized from the present nucleic acid by utilizing a method well known in the technical field. For example, host cells are transformed with a vector containing DNA encoding the present peptide, the thus obtained transformed cells are then cultured, and a peptide of interest is then harvested from the culture, so as to obtain the peptide of the present invention.

The peptide of the present invention may have a cyclic structure. The cyclic structure may not only be a structure in which the N-terminus is connected with the C-terminus, but may also be a structure in which any terminus is cyclized with a side chain or side chains are cyclized with each other. A person skilled in the art could readily produce such a peptide having a cyclic structure. Moreover, it is also possible to ask the production of such a cyclic peptide to an enterprise or the like which undertakes the production thereof.

Further, the embodiment of the present invention includes a peptide complex comprising a plurality of the peptides of the present invention. In this peptide complex, the peptides may directly bind to one another, or may indirectly bind to one another via a linker and the like. For example, PEG having a plurality of arms is used as a linker, and peptides are allowed to bind to individual arms of the PEG via maleimide groups or the like, so that a peptide complex comprising multiple peptides can be produced.

A third embodiment of the present invention relates to a detection reagent containing the peptide of the present invention as an active ingredient, which is used for detecting a metal surface of a device (in particular, a stent, a coil for embolization, etc.), wherein the detection reagent comprises the peptide or the peptide complex of the present invention (hereinafter referred to as "the detection reagent of the present invention").

More specifically, the detection reagent for detecting a metal surface of a device made of nickel and/or titanium may comprise one or more peptides according to the first embodiment and/or one or more peptide complexes comprising multiple peptides according to the first embodiment. Moreover, the detection reagent for detecting a metal surface of a device made of platinum and/or tungsten may comprise one or more peptides according to the second embodiment and/or one or more peptide complexes comprising multiple peptides according to the second embodiment.

The aforementioned detectable labeling substance may bind to the peptide of the present invention comprised in the detection reagent of the present invention.

The detection reagent of the present invention is administered into the blood flow of a subject, in which a stent or a coil for embolization is placed, and the peptide or the peptide complex of the present invention is allowed to bind to a metal exposed portion of the stent or the coil for embolization. Thereafter, signals obtained from the bound peptide or peptide complex (i.e., fluorescent signals, radioactive signals, or MRI signals) are detected and are then quantified, so that the exposed portion (i.e., a portion that is not covered with endothelial cells or the like) of the stent or the coil for embolization placed in the body can be imaged and quantified. The form of the detection reagent of the present invention may be either powders obtained by freeze-drying the peptide of the present invention, or a liquid in which the peptide of the present invention is dissolved. Thus, the detection reagent of the present invention may have any form, as long as it is suitable for administration to a subject. Furthermore, the detection reagent of the present invention may also comprise pharmaceutically acceptable buffers, preservatives, sterilized water for infection, chelating agents and pH adjusters, as necessary, in addition to the peptide of the present invention used as an active ingredient.

The detection reagent of the present invention may have the form of a kit, and may be included in a vessel or a pack, together with administration instructions. When the detection reagent of the present invention is suppled in the form of a kit, a plurality of constitutional components of the detection reagent (e.g., the peptide of the present invention, sterilized water, a buffer, and the like) may be included in each different vessels, and the constitutional components may be then mixed with one another immediately before the use. The peptide of the present invention and other constitutional components, which are included in a kit, need to be placed in a vessel made of a material in which the peptide or the constitutional components can maintain activity for a long period of time. When the vessel is an ampoule, the constitutional components may be placed therein together with neutral inert gas such as nitrogen gas. The material of the ampoule is favorably a glass, an organic polymer such as polycarbonate or polystyrene, or a ceramic. A metal that may adsorb the peptide of the present invention should be avoided.

A fourth embodiment of the present invention relates to a metallic device to which the peptide of the present invention binds. This metallic device is preferably a device made of nickel and/or titanium, or a device made of platinum and/or tungsten, and is particularly preferably a medical device (a stent, a coil for embolization, etc.).

A fifth embodiment of the present invention relates to an antibody reacting against the peptide of the present invention (hereinafter referred to as "the antibody of the present invention"). The antibody reacting against the peptide of the present invention as an antigen can be obtained by a known method. The antibody of the present invention may be either a monoclonal antibody or a polyclonal antibody.

The presence of the peptide of the present invention can also be detected by indirectly labeling the peptide via a labeling substance-binding antibody, and then obtaining signals obtained from the labeling substance. This method is effective, when a stent or a coil for embolization, to which the peptide of the present invention has previously bound, is placed in a body and a portion of the stent or the coil for embolization that is not covered with endothelial cells or the like is then detected.

A sixth embodiment of the present invention relates to a method for detecting a metal surface of a medical device (i.e., a metal surface that is not covered with endothelial cells or the like), which comprises: administering the detection reagent of the present invention, and/or an antibody reacting against a peptide comprised in the detection reagent, to which a detectable labeling substance binds, to a subject in whom a medical device (e.g., a stent or a coil for embolization) has been placed in the blood vessel thereof; and then detecting labeled signals obtained from the site in which the medical device is placed.

As mentioned above, signals obtained from the labeling substance can be detected and quantified by a PET (positron emission tomography) method or a SPECT (single photon emission computed tomography) method. In addition, such signals can also be detected or quantified by an MRI method using a gadolinium contrast agent or a superparamagnetic iron oxide nanoparticle contrast agent.

More specifically, when the medical device is made of nickel and/or titanium, the above-described "the detection reagent of the present invention" desirably comprises one or more peptides according to the first embodiment, and/or one or more peptide complexes comprising multiple peptides according to the first embodiment. On the other hand, when the medical device is made of platinum and/or tungsten, the above-described "the detection reagent of the present invention" desirably comprises one or more peptides according to the second embodiment, and/or one or more peptide complexes comprising multiple peptides according to the second embodiment.

The disclosures of all publications cited in the present description are incorporated herein by reference in their entirety. In addition, throughout the present description as a whole, when singular terms such as "a," "an," and "the" are used, these terms include not only single items but also multiple items, unless otherwise clearly specified.

Hereinafter, the present invention will be further described in the following examples. However, these examples are only illustrative examples of the embodiments of the present invention, and thus, are not intended to limit the scope of the present invention.

### Examples

### 1. Preparation of the peptide of the present invention

### Phage Display Method

According to a phage display method, a peptide having high affinity for a stent or a coil was searched. Targeting a peptide consisting of 12 residues, Ph.D.-12 Phage Display Peptide Library Kit (New England Biolabs) was used.

A stent (Neuroform EZ, Stryker) or a coil (GDC 10-Soft coil, Boston Scientific) used as a target was sterilized with 70% ethanol, and was then heated to 37°C in PBS. A phage library (1.2 x 10¹⁰ pfu) was exposed to the stent or the coil for 15 minutes, and the stent or the coil was then washed with a 0.1% Tween 20 solution (in PBS) and PBS to remove the phage library that was non-specifically adsorbed on the stent or the coil. The phage library that was specifically adsorbed on the stent or the coil was eluted with a Glycine-HCl solution with pH = 2.2 to obtain a phage library of interest. Thereafter, the phage pool was sufficiently amplified using *Escherichia coli* (ER2738). The present step was defined as one panning, and five pannings were carried out in total, so as to obtain a phage library having high affinity for the stent or the coil.

DNA was extracted from the obtained phage library, using DNA Extraction Kit (Qiagen). Sequencing was outsourced to Eurofins Genomics K. K.

### Peptide Adsorption Experiment

Peptides were synthesized based on the sequences obtained by phage display (commissioned synthesis, Sigma-Aldrich, GenScript Japan, Inc.), and fluorescein isothiocyanate (FITC) was then allowed to bind to the N-terminus of each peptide. Into the C-terminus, cysteine was introduced. This is because chemical modification was performed on polymers, etc.

The amino acid sequences of peptides selectively binding to the stent are shown in Table 1, whereas the amino acid sequences of peptides selectively binding to the coil for embolization are shown in Table 2.

**[Table 1]**

| Peptide | Amino acid sequence | |
|---|---|---|
| YT01 | MKAHHSQLYPRH | (SEQ ID NO:1) |
| YT02 | HRPYLQSHHAKM | (SEQ ID NO:2) |
| YT11 | HAPDTIKRSLAM | (SEQ ID NO:3) |
| YT12 | DKDVTHFLERTR | (SEQ ID NO:4) |
| YT13 | NHHMMPAWNVKH | (SEQ ID NO:5) |
| YT14 | NLGAEPGTPYLV | (SEQ ID NO:6) |
| YT15 | YQPAREHRVPAG | (SEQ ID NO:7) |
| YT16 | TNNNTPSQMGLS | (SEQ ID NO:8) |
| YT17 | FQQASQARPSSP | (SEQ ID NO:9) |
| YT18 | ADSNHAYERDSV | (SEQ ID NO:10) |
| YT19 | LGTKPALSPTNF | (SEQ ID NO:11) |
| YT20 | TLNVPPAKRSLS | (SEQ ID NO:12) |
| YT21 | ATHGPKQVSSWT | (SEQ ID NO:13) |

| | | |
|---|---|---|
| * YT02 and YT04 are inverted sequences of YT01 and YT03, respectively. * FITC is introduced into N-terminus, whereas a spacer + cysteine (GGGC) are introduced into C-terminus. | | |

**[Table 2]**

| Peptide | Amino acid sequence | |
|---|---|---|
| YT05 | AHNHTPIKQKYL | (SEQ ID NO:14) |
| YT06 | LYKQKIPTHNHA | (SEQ ID NO:15) |
| YT07 | LTPHKHHKHLHA | (SEQ ID NO:16) |
| YT08 | AHLHKHHKHPTC | (SEQ ID NO:17) |
| YT09 | SAVQWFELNTHA | (SEQ ID NO:18) |
| YT10 | AHTNLEFWQVAS | (SEQ ID NO:19) |

| | | |
|---|---|---|
| * FITC is introduced into N-terminus, whereas a spacer + cysteine (GGGC) are introduced into C-terminus. | | |

FITC was introduced into the N-terminus of the prepared peptide, whereas a spacer and cysteine (GGGC) were introduced into the C-terminus thereof. The stent or the coil was immersed in a 0.5 mM peptide solution (in PBS) (37°C, 30 minutes), so that the peptide was allowed to bind to the stent or the coil. After that, the stent or the coil was sufficiently rinsed with PBS. Thereafter, in order to examine the presence or absence of the binding the peptide, the stent or the coil was observed under an upright fluorescence microscope.

As a result, it was confirmed that the peptides shown in Table 1 and Table 2 each bound to the surfaces of the stent and the coil.

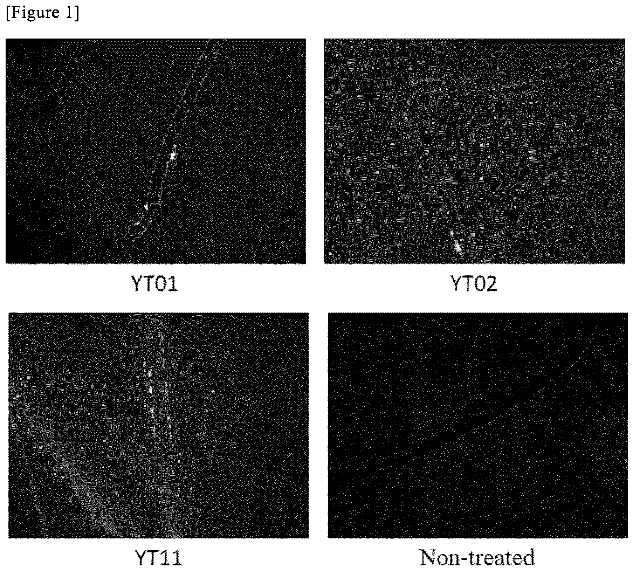

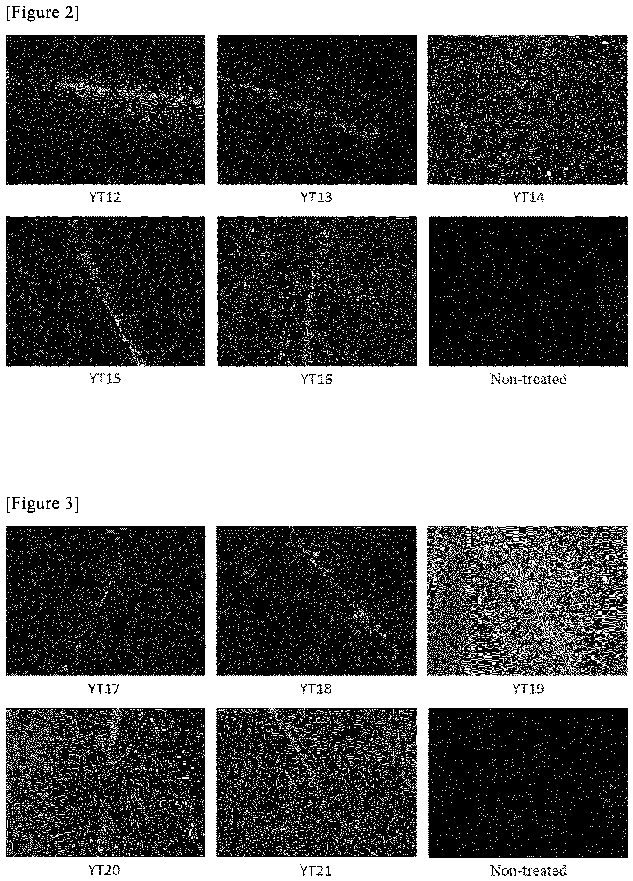

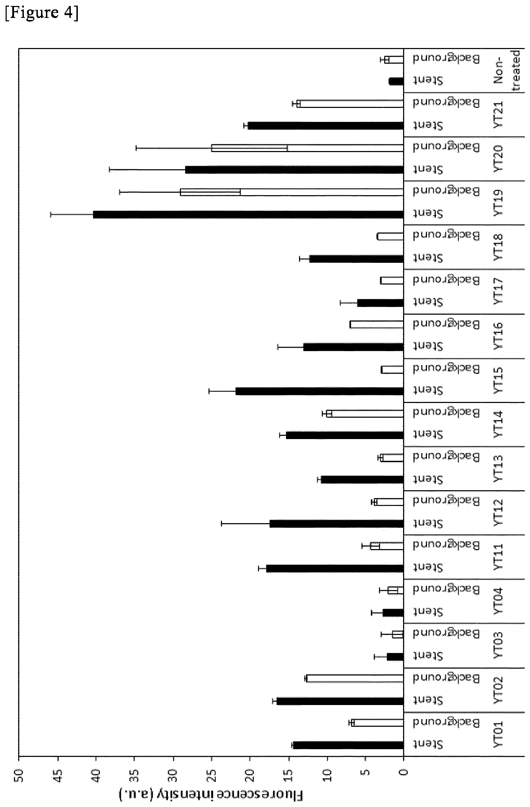

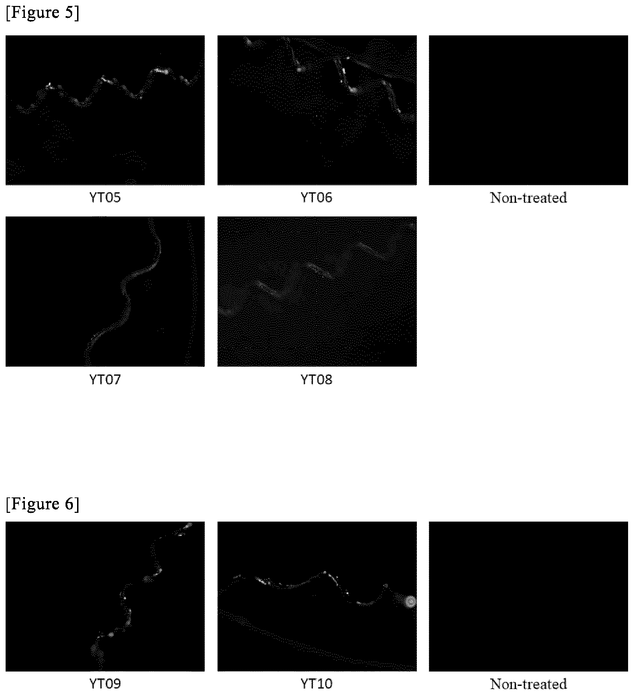

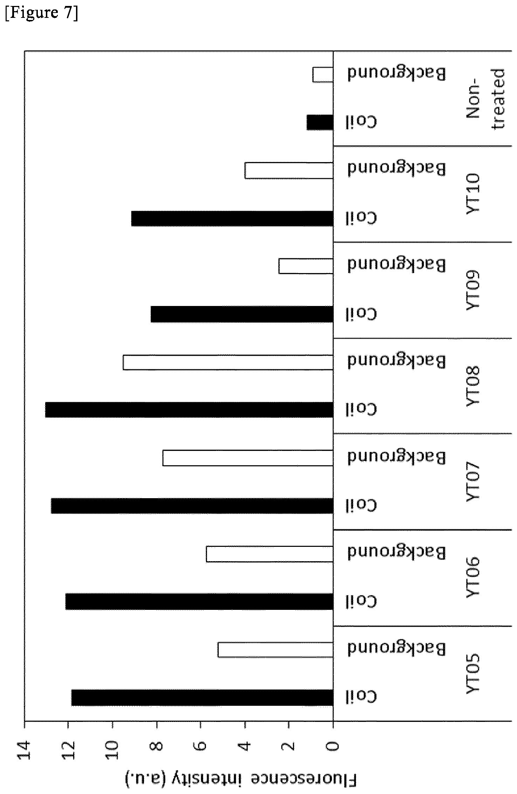

Figure 1 to Figure 3 include photomicrographs, in each of which fluorescence emitted from the peptide binding to the surface of the stent is observed. In addition, the fluorescence intensities of fluorescence states observed in each of Figure 1 to Figure 3 are summarized in Figure 4. Figure 5 and Figure 6 include photomicrographs, in each of which fluorescence emitted from the peptide binding to the surface of the coil for embolization is observed. In addition, the fluorescence intensities of fluorescence states observed in each of Figure 5 and Figure 6 are summarized in Figure 7. The results shown in Figure 4 and Figure 7 were obtained by analyzing the fluorescence images using ImageJ (National Institutes of Health), and digitizing the fluorescence intensity from the stent or the coil and the fluorescence intensity from background fluorescence.

It was found that all of the peptides indicated herein specifically bound to the surface of the stent or the coil. From the obtained fluorescence intensities, the affinity of the peptide can be evaluated. The fact that the background shows a great value demonstrates dissociation of the peptide from the metal surface. The affinity is different depending on the sequence of the peptide.

### Experiment Regarding Adsorption of PEG-Peptide Conjugate

In actual use, the use of a single peptide is possible. However, taking into consideration administration of the peptide into blood, it has been predicted that a peptide complex formed by depositing peptides onto a branched polymer such as, for example, PEG would efficiently bind to a stent or a coil. Hence, peptides were allowed to bind to the tips of PEG having 4 arms (branches) to produce a peptide complex (a PEG-peptide conjugate).

Specifically, the peptides were allowed to react with 4-branched PEG (SUNBRIGHT PTE-200MA, NOF corporation) having maleimide groups at the termini thereof and having a PEG chain with a molecular weight of 20,000 Da per branch, at a molar ratio of 1 : 4 (room temperature, overnight). That is, the peptides were allowed to bind to the 4-branched PEG according to a thiol-maleimide reaction via the thiol of the C-terminal cysteine of the peptide. A stent or a coil was immersed in a 0.5 mM PEG-peptide solution (in PBS) (37°C, 30 minutes), and was then sufficiently rinsed with PBS, which was then observed under a fluorescence microscope.

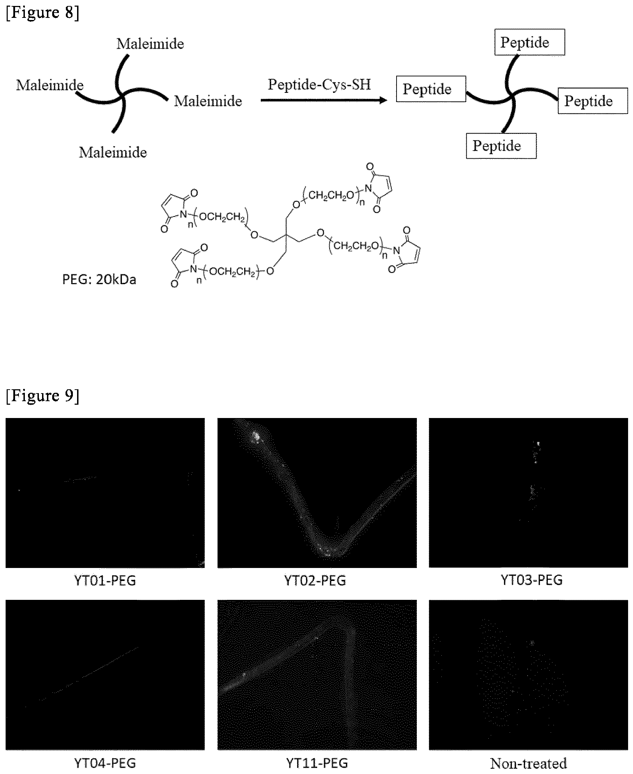

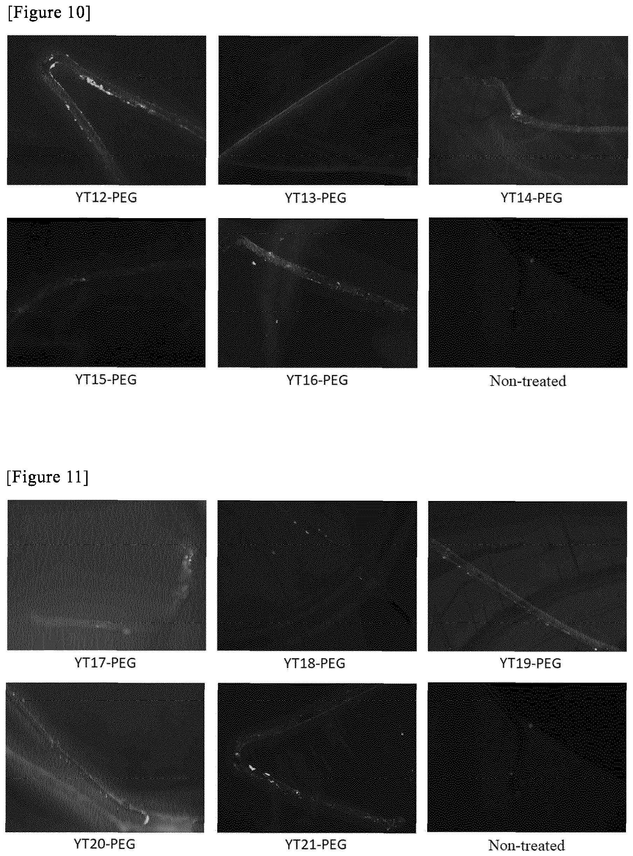

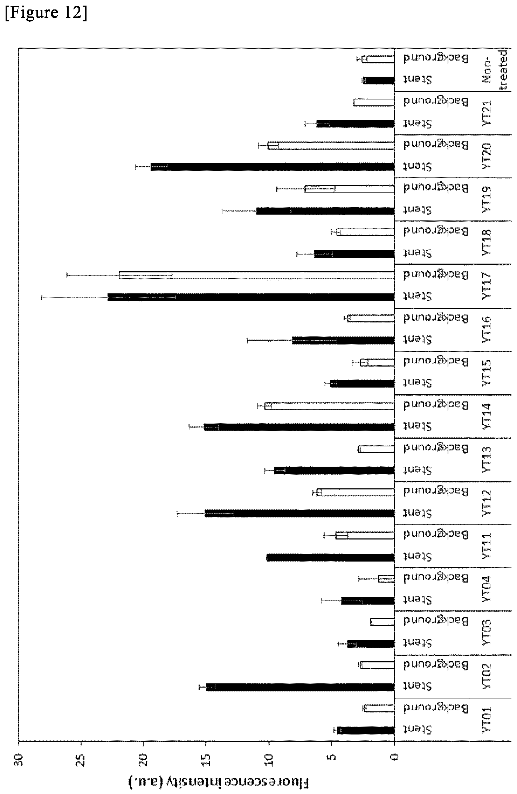

Figure 9 to Figure 11 include photomicrographs, in each of which fluorescence emitted from the peptide complex binding to the surface of the stent is observed. In addition, the fluorescence intensities of fluorescence states observed in each of Figure 9 to Figure 11 are summarized in Figure 12.

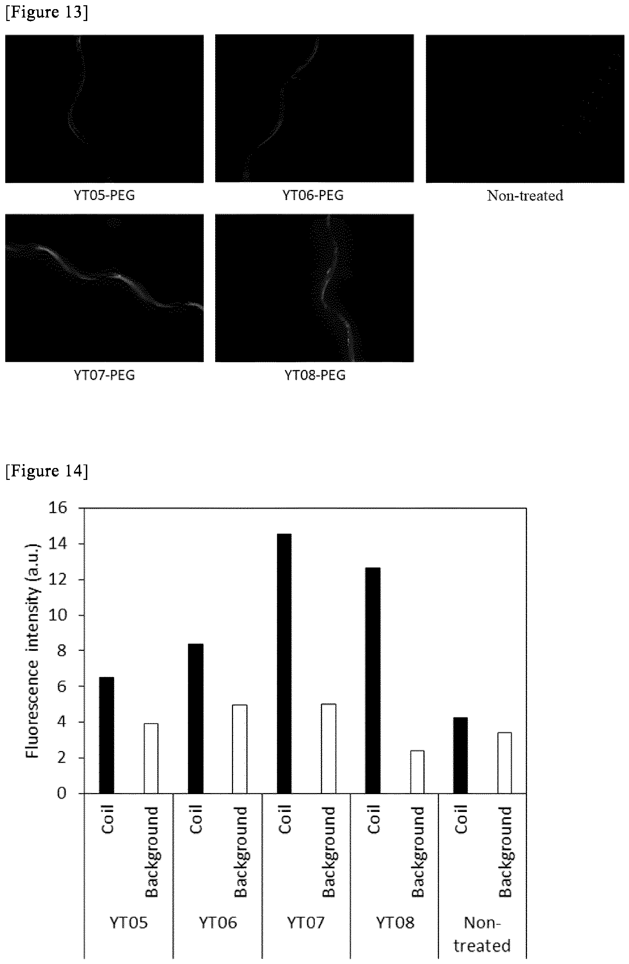

Figure 13 includes photomicrographs, in each of which fluorescence emitted from the peptide complex binding to the surface of the coil for embolization is observed. In addition, the fluorescence intensities of fluorescence states observed in Figure 13 are summarized in Figure 14. The method of digitizing the fluorescence intensity is as described above.

Regarding the peptide complex as well, fluorescence is observed from the surface of the stent or the coil, and the influence of the formation of a conjugate with 4-branched PEG is not found.

### Experiment Regarding Adsorption on Stent in Plasma

Next, in order to analyze the binding ability of the peptide of the present invention to a stent existing in plasma, whether or not the peptide of the present invention binds to a stent that has been allowed to come into contact with plasma was examined.

That is, a stent was immersed in human plasma (37°C, 30 minutes), and was then removed from the plasma. The stent was then immersed in each peptide solution (0.5 mM) and a PEG-peptide conjugate solution (0.5 mM) (37°C, 30 minutes). Thereafter, the stent was sufficiently rinsed with PBS, and was then observed under a fluorescence microscope.

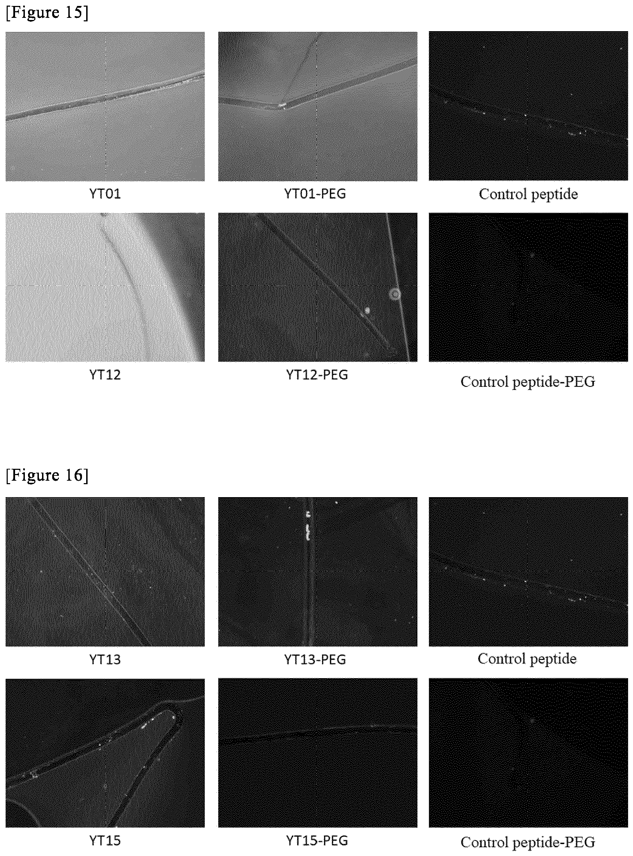

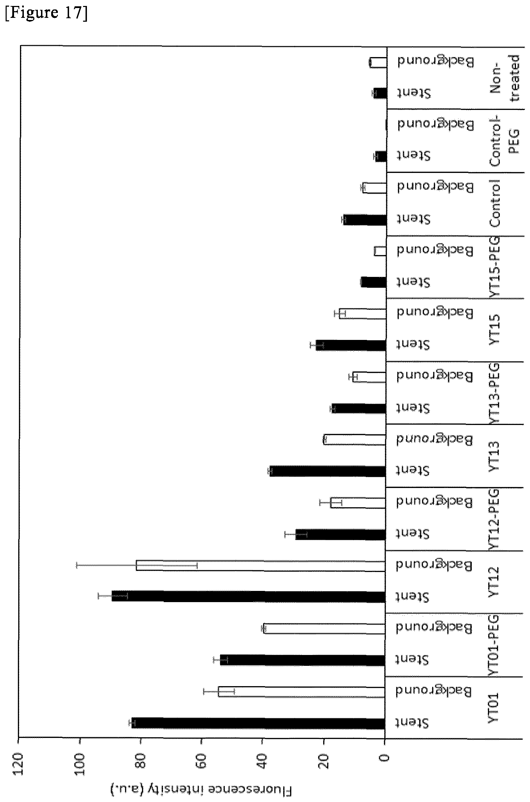

Figure 15 and Figure 16 show the fluorescence microscopic images of the stents, and the observed fluorescence intensities are summarized in Figure 17. The method of digitizing the fluorescence intensity is as described above.

The peptides and the PEG-peptide conjugates shown herein specifically bound to the surface of the stent even in human plasma.

### Industrial Applicability

The peptide of the present invention selectively binds to the surface of a medical device such as a stent or a coil for embolization. Accordingly, the peptide of the present invention is useful for evaluation of the surface of a metallic medical device placed in the body, and thus, it is expected that the present peptide will be utilized in the medical field.

## Claims

1. A peptide consisting of an amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15.

2. A peptide of the following (a) or (b), which selectively binds to nickel and/or titanium:
(a) a peptide consisting of an amino acid sequence comprising a substitution, deletion, insertion and/or addition of one or several amino acids, with respect to the amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15, or
(b) a peptide consisting of an amino acid sequence having a sequence identity of 90% or more to the amino acid sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15.

3. The peptide according to claim 1 or claim 2, to which a detectable labeling compound binds.

4. A peptide consisting of an amino acid sequence as set forth in SEQ ID NO: 16, SEQ ID NO: 17, SEQ ID NO: 18, SEQ ID NO: 19, SEQ ID NO: 20, or SEQ ID NO: 21.

5. A peptide of the following (a) or (b), which selectively binds to platinum and/or tungsten:
(a) a peptide consisting of an amino acid sequence comprising a substitution, deletion, insertion and/or addition of one or several amino acids, with respect to the amino acid sequence as set forth in SEQ ID NO: 16, SEQ ID NO: 17, SEQ ID NO: 18, SEQ ID NO: 19, SEQ ID NO: 20, or SEQ ID NO: 21, or
(b) a peptide consisting of an amino acid sequence having a sequence identity of 90% or more to the amino acid sequence as set forth in SEQ ID NO: 16, SEQ ID NO: 17, SEQ ID NO: 18, SEQ ID NO: 19, SEQ ID NO: 20, or SEQ ID NO: 21.

6. The peptide according to claim 4 or claim 5, to which a detectable labeling compound binds.

7. A peptide complex comprising two or more peptides selected from the group of the peptides according to claim 1, claim 2, and claim 3.

8. A peptide complex comprising two or more peptides selected from the group of the peptides according to claim 4, claim 5, and claim 6.

9. An antibody against the peptide according to any one of claim 1, claim 2, claim 4, or claim 5.

10. A nucleic acid encoding the peptide according to any one of claim 1, claim 2, claim 4, or claim 5.

11. A detection reagent for detecting a metal surface of a device made of nickel and/or titanium, wherein the detection reagent comprises the peptide selected from the group of the peptides according to claim 1, claim 2, and claim 3, and/or the peptide complex according to claim 7.

12. A detection reagent for detecting a metal surface of a device made of platinum and/or tungsten, wherein the detection reagent comprises one or more peptides selected from the group of the peptides according to claim 4, claim 5, and claim 6, and/or the peptide complex according to claim 8.

13. A metallic device, to which the peptide according to any one of claims 1 to 6 or the peptide complex according to claim 7 or 8 binds.
